# EUROPÄISCHE PATENTSCHRIFT

(11) **EP 2 914 141 B1**
(45) Veröffentlichungstag und Bekanntmachung des Hinweises auf die Patenterteilung: **17.03.2021**
(21) Anmeldenummer: 13773707.8
(22) Anmeldetag: 02.10.2013
(51) Int. Cl.: A43B 7/14, A61F 5/14

(54) **VISCOELASTISCHES ELEMENT**
VISCOELASTIC ELEMENT
ÉLÉMENT VISCOÉLASTIQUE

(30) Priorität: 30.10.2012 DE 102012021696
(43) Veröffentlichungstag der Anmeldung: 09.09.2015
(73) Patentinhaber: Bauerfeind AG, 07937 Zeulenroda-Triebes (DE)
(72) Erfinder: JAEGER, Thorsten, 08527 Plauen (DE); PANZER, Dominique, 07937 Zeulenroda-Triebes (DE)
(74) Vertreter: Wohlfahrt, Jan Günther
(86) Internationale Anmeldenummer: PCT/EP2013/070557
(87) Internationale Veröffentlichungsnummer: WO 2014/067739

(56) Entgegenhaltungen:
- WO-A2-2007/149429
- CN-U- 201 813 948
- US-A1- 2001 045 028
- US-A1- 2005 039 349

## Beschreibung

Die Erfindung betrifft Elemente für zumindest teilweise viscoelastische Medizinprodukte oder für zumindest teilweise viscoelastische orthopädische Hilfsmittel, zumindest teilweise viscoelastische Medizinprodukte oder zumindest teilweise viscoelastische orthopädische Hilfsmittel, die solche Elemente enthalten oder aus ihnen bestehen, und die Verwendung solcher Elemente.

Viscoelastische orthopädische Hilfsmittel sind beispielsweise Fersenkissen. Diese sind häufig aus einem einzigen Material, also einer einzigen Komponente, gefertigt oder haben an einem definierten Bereich in diese Komponente ein weiteres weicheres Material integriert, so dass zum Beispiel ein Fersensporn entlastet wird. Hierdurch ist eine indikationsbezogene Entlastung aber nur eingeschränkt möglich.

Auch andere viscoelastische orthopädische Hilfsmittel und viscoelastische Medizinprodukte, wie zum Beispiel Pelotten sind meistens aus einem einzigen Material gefertigt.

Ein elastisches Element nach dem Obergriff des Anspruchs 1 wird in CN 201 813 948 U offenbart.

Der Erfindung liegt das technische Problem zugrunde, neuartige Elemente für viscoelastische Medizinprodukte oder für teilweise viscoelastische orthopädische Hilfsmittel bereitzustellen, die gegenüber dem Stand der Technik verbessert sind. Insbesondere sollen die Elemente eine verbesserte indikationsbezogene Stützung, Entlastung, Stimulation, Bewegungsführung und/oder Propriozeption des anliegenden Körperbereichs, beispielsweise der Fußsohle, der Ferse oder des Knies ermöglichen.

Das der Erfindung zugrunde liegende technische Problem wird gelöst durch die Bereitstellung eines viscoelastischen Elements nach Anspruch 1.

Es zeigte sich überraschender Weise, dass die Viscoelastizität eines Elements durch den erfindungsgemäßen schichtförmigen Aufbau aus mindestens einer ersten und einer zweiten Komponente unterschiedlicher Härte und durch die Ausgestaltung der Oberfläche der ersten Komponente, die mit der zweiten Komponente verbunden ist, mit einer Vielzahl von Erhebungen, eingestellt werden kann. Es zeigte sich insbesondere, dass in vorteilhafter Weise durch den erfindungsgemäßen schichtförmigen Aufbau und durch die Höhe, die Anzahl und die spezifische Form der Erhebungen Flächenzonen in dem Element mit unterschiedlicher Härte beziehungsweise Viscosizität erzeugt werden können.

Somit können Zonen unterschiedlicher Viscoelastizität indikationsbezogen und positionsgenau erzeugt werden, wobei die Viscoelastizität einer Zone im Bereich der verwendeten Materialien frei gewählt werden kann und das Element selbst bei Verwendung von nur zwei Komponenten ohne weitere Komponente mehr als zwei unterschiedliche Viscoelastizitäten aufweisen kann.

Die Erhebungen, zum Beispiel Zapfen und/oder Wellen, können auch in vorteilhafter Weise als Stimulationspunkte wirken, die die anliegende Körperoberfläche gezielt durch Druck stimulieren.

Darüber hinaus wird die Grenzfläche zwischen der ersten Komponente und der zweiten Komponente durch die Erhebungen erhöht, so dass die beiden Komponenten besser miteinander verbunden werden können beziehungsweise verbunden sind. Auch muss nicht zwingend ein weicheres Material in ein Grundmaterial integriert werden, sondern es kann eine einfachere schichtförmige Kombination der Materialien verwendet werden.

Da die beiden schichtförmigen Komponenten miteinander über jeweils einer ihrer Oberflächen verbunden werden und die erste Komponente an dieser Oberfläche Erhebungen aufweist, ist vorgesehen, dass die Oberfläche der zweiten Komponente an dieser Position eine dementsprechende Vertiefung aufweist, dass also die beiden Komponenten an ihren entsprechenden Oberflächen nach einen Schloss-Schlüssel-Prinzip miteinander verbunden sind beziehungsweise dass die Oberfläche der zweiten Komponente ein negativ der Oberfläche der ersten Komponente ist.

Die Oberflächen der ersten und der zweiten Komponente, die nicht mit der jeweils anderen Komponente verbunden sind, sind glatt.

In einer bevorzugten Ausführungsform haben die erste und die zweite Komponente eine unterschiedliche Shorehärte.

In einer bevorzugten Ausführungsform bestehen die erste und die zweite Komponente aus Materialien mit unterschiedlicher Shorehärte. In einer bevorzugten Ausführungsform bestehen die erste und die zweite Komponente aus unterschiedlichen Materialien mit unterschiedlicher Shorehärte.

Die Shorehärte einer Komponente kann durch die Wahl der jeweiligen Materialien und/oder durch den Aufbau des Materials bestimmt werden. Ohne an die Theorie gebunden zu sein können zum Beispiel die erste und die zweite Komponente auch aus dem gleichen Material, einem im Wesentlichen gleichen Material oder einem ähnlichen Material bestehen, wobei sie trotzdem durch die jeweilige strukturelle Beschaffenheit des Materials eine unterschiedliche Shorehärte aufweisen.

In einer bevorzugten Ausführungsform bestehen die erste und die zweite Komponente aus viscoelastischen Materialien.

Einem Fachmann sind geeignete Materialien, insbesondere viscoelastische Materialein, bekannt. Er kann auch ohne Weiteres die Shorehärte der Materialien bestimmen. Geeignete Materialien sind bevorzugt viscoelastisch.

Geeignete Materialien sind beispielsweise solche, die in viscoelastische Medizinprodukten oder in viscoelastischen orthopädische Hilfsmitteln aus dem Stand der Technik verwendet werden, beispielsweise bei Pelotten, Einlegesohlen oder Fersenkissen.

Geeignete Materialien sind zum Beispiel thermoplastische und duroplastische Elastomere.

Die Erhebungen können jegliche gewünschte Form haben.

In einer bevorzugten Ausführungsform sind die Erhebungen zapfenförmig oder wellenförmig.

In einer bevorzugten Ausführungsform sind die Erhebungen zapfenförmig. In einer bevorzugten Ausführungsform weist die erste Komponente eine Vielzahl von Zapfen als Erhebungen auf.

Unter Zapfen wird im Zusammenhang mit der vorliegenden Erfindung ein hervorstehendes Stück zur Verbindung der zwei Komponenten verstanden. Ein Zapfen ist bevorzugt derart ausgestaltet, dass Länge und Breite des Zapfens ähnlich sind, dass insbesondere der Zapfen in einem Querschnitt höchstens 5 mal länger ist als er breit ist. Ein Zapfen kann jegliche geeignete Form haben, beispielsweise die Form eines Würfels, eines Quaders, einer Pyramide, eines Kegels, einer Kugel oder eines Zylinders. Der Querschnitt eines Zapfens kann jegliche zweidimensionale Form haben, beispielsweise eine runde, ovale, ellipsoide oder mehreckige, beispielsweise viereckige, insbesondere quadratische, fünfeckige, sechseckige, achteckige oder sternenförmige, Form haben. Der Querschnitt eines Zapfens kann über die Länge des Zapfens hinweg gleich sein oder sich verändern, insbesondere von der Grundoberfläche aus gesehen verjüngen.

Natürlich kann die erste Komponente auch Zapfen unterschiedlicher Form aufweisen, da sich die Viscoelastizität auch durch die Form der Zapfen beeinflussen lässt.

Natürlich können auch Zapfen und Wellen als Erhebungen kombiniert werden.

Bevorzugt wird die Viscoelastizität einer Zone durch die Höhe der dortigen Erhebungen der ersten, härteren Komponente bestimmt, also durch die Länge der Erhebungen von der Grundfläche aus gesehen. Je höher die Erhebungen sind umso härter wird die entsprechende Zone.

Alternativ wird die Viscoelastizität einer Zone durch die Verteilung der dortigen Erhebungen der ersten, härteren Komponente bestimmt. Je mehr Erhebungen pro Flächeneinheit vorhanden sind umso härter wird die entsprechende Zone.

Natürlich ist in vorteilhafter Weise auch eine Kombination von Höhe und Verteilung der Erhebungen zum Einstellen der gewünschten Viscoelastizität in einer Zone möglich.

In einer bevorzugten Ausführungsform bestehen die erste Komponente und/oder die zweite Komponente aus einem durchsichtigen oder durchscheinenden Material. Dadurch ist der erfindungsgemäße Aufbau des Elements sichtbar und ein Fachmann kann die genaue Position der Zonen unterschiedlicher Viscoelastizität erkennen.

In einer bevorzugten Ausführungsform besteht das Element aus den zwei schichtförmigen Komponenten.

In einer alternativen Ausführungsform weist das Element mindestens eine dritte Komponente auf.

Die dritte Komponente kann ebenfalls schichtförmig sein und als weitere Schicht auf der ersten Komponente oder auf der zweiten Komponente eine weitere Schicht des Elements bilden. Das gleiche gilt für eine vierte Komponente und weitere Komponenten.

In einer bevorzugten Ausführungsform ist die dritte Komponente eine Schicht, die die erste Komponente oder die zweite Komponente abdeckt. In einer bevorzugten Ausführungsform ist die dritte Komponente eine Folie, ein Stoff oder ein Vlies. In einer besonders bevorzugten Ausführungsform ist die dritte Komponente eine Folienkaschierung.

In einer bevorzugten Ausführungsform ist die dritte Komponente eine Schicht, die die erste Komponente abdeckt und die vierte Komponente eine Schicht, die die zweite Komponente abdeckt. In einer bevorzugten Ausführungsform sind die dritte und die vierte Komponente eine Folie, ein Stoff oder ein Vlies. In einer besonders bevorzugten Ausführungsform sind die dritte und die vierte Komponente eine Folienkaschierung.

Eine dritte schichtförmige Komponente kann insbesondere dazu dienen, eine angenehme, beispielsweise nicht klebrige oder kühle, Kontaktfläche des Elements zu dem entsprechenden Körperteil des Patienten, beispielsweise zur Fußsohle, zu bilden.

Die dritte Komponente kann aber auch in einem Teilbereich der ersten Komponente und/oder der zweiten Komponente durch die durch die erste Komponente und/oder die zweite Komponente gebildete Schicht hindurchreichen oder in die durch die erste Komponente und/oder zweite Komponente gebildete Schicht eingebettet sein. Das gleiche gilt für eine vierte Komponente und weitere Komponenten.

In einer bevorzugten Ausführungsform kann das Element, beispielsweise in Form einer Pelotte, einer Einlegesohle oder eines Fersenkissens, fertig ausgeformt vorliegen. Alternativ kann aber auch vorgesehen sein, dass das Element nachbearbeitbar ist, so dass beispielsweise ein Orthopädie-Schuhtechniker ein erfindungsgemäßes Element, beispielsweise eine erfindungsgemäße Schuheinlage oder ein erfindungsgemäßes Fersenkissen noch individuell in der Form verfeinern kann.

In einer bevorzugten Ausführungsform ist das Element ein Element für ein orthopädisches Medizinprodukt, also insbesondere Bestandteil eines orthopädischen Medizinprodukts. In einer alternativen Ausführungsform ist das Element ein orthopädisches Medizinprodukt.

In einer bevorzugten Ausführungsform ist das Element Bestandteil einer Einlegesohle oder eines Fersenkissens.

In einer bevorzugten Ausführungsform ist das Element eine Einlegesohle oder ein Fersenkissen.

In einer bevorzugten Ausführungsform handelt es sich um eine viscoelastische Einlegesohle oder um ein viscoelastisches Fersenkissen.

In einer bevorzugten Ausführungsform bildet die erste Komponente den unteren Teilbereich der Einlegesohle oder des Fersenkissens und die zweite Komponente bildet den oberen Teil der Einlegesohle oder des Fersenkissens. In einer alternativen Ausführungsform bildet die erste Komponente den oberen Teilbereich der Einlegesohle oder des Fersenkissens und die zweite Komponente bildet den unteren Teil der Einlegesohle oder des Fersenkissens.

In einer bevorzugten Ausführungsform ist das Element Bestandteil einer Pelotte. In einer bevorzugten Ausführungsform ist das Element eine Pelotte, bevorzugt eine viscoelastische Pelotte, insbesondere ringförmige oder wellenförmige Pelotte.

Kniegelenksorthesen mit einem ringförmigen oder halbringförmigen Pelottenkissen, das die Patella des Kniegelenks im angelegtem Zustand der Orthese umschließt, sind bekannt. Solche Orthesen können bekanntermaßen als strumpf- oder schlauchförmige elastische Gestricke ausgebildet sein. In dem Gestrick ist im Bereich der Patella eine Pelotte eingesetzt. Die Pelotte stützt die Patella und fixiert diese vor allem in Verbindung mit der durch das elastische Gestrick vermittelten Druckbeaufschlagung in physiologisch korrekter Position im Gelenk. Bekannte Pelotten sind aus einem elastischen Werkstoff wie Silikonkautschuk oder Polyurethan oder ähnlichen Werkstoffen gefertigt.

Die Pelotte ist in ihrer Grundform (Pelottenbasis) besonders ringförmig ausgebildet und umschließt die Patella. In einer anderen besonderen Ausgestaltung ist die Pelotte hufeisenförmig, das heißt halbringförmig, ausgebildet und umgreift die Patella zumindest distal. Die neuartige Pelotte wird in an sich bekannter Weise bevorzugt in einer elastischen Gestrickorthese eingesetzt. Über die Gestrickorthese wird die Pelotte an das Kniegelenk im Bereich der Patella angepresst. Die distalen Vorsprünge entfalten ihre Wirkung vor allem in Verbindung mit dem elastischen Grundgestrick der Bandage und treten durch die Bewegung mit diesem in Wechselwirkung.

Durch den erfindungsgemäßen Aufbau kann die Pelotte in vorteilhafter Weise Zonen unterschiedlicher Härte aufweisen, so dass sie unterschiedlich stark an das Kniegelenk im Bereich der Patella anpresst.

Gegenstand der Erfindung ist auch ein Medizinprodukt, insbesondere orthopädisches Medizinprodukt oder orthopädisches Hilfsmittel, enthaltend ein erfindungsgemäßes Element. Gegenstand der Erfindung ist auch ein Medizinprodukt, insbesondere orthopädisches Medizinprodukt oder orthopädisches Hilfsmittel, bestehend aus einem erfindungsgemäßen Element.

Bevorzugt handelt es sich bei dem Medizinprodukt oder dem orthopädisches Hilfsmittel um eine Einlegesohle, ein Fersenkissen oder eine Pelotte.

Gegenstand der Erfindung ist insbesondere auch Einlegesohlen und Fersenkissen, die erfindungsgemäße Elemente enthalten.

Gegenstand der Offenbarung ist auch die therapeutische und/oder prophylaktische Verwendung der Einlegesohlen und Fersenkissen, die erfindungsgemäße Elemente enthalten, zur physiologischen Dämpfung und Stützung des Fußes im Fersen-, Mittel- und Vorfußbereich. Im Speziellen zur Behandlung eines Fersensporns, Arthrose der Beingelenke, Achillodynie, Haglundferse, Beinlängendifferenz und Rückfußbeschwerden.

Gegenstand der Erfindung ist auch eine Kniegelenksorthese oder -bandage, welche die erfindungsgemäße Pelotte enthält. Diese ist besonders als Gestrickorthese mit eingelegter erfindungsgemäßer Pelotte ausgestaltet.

Gegenstand der Erfindung ist auch die Verwendung der erfindungsgemäßen Pelotte mit den daran ausgebildeten Vorsprüngen zur Verbesserung und/oder zur Sicherung der Positionierung der Gelenksorthese am Körpergelenk.

Gegenstand der Offenbarung ist auch die therapeutische und/oder prophylaktische Verwendung der erfindungsgemäßen Pelotte in einer Kniegelenksorthese zur Unterstützung des kongruenten Schlusses zwischen den infrapatellaren Gelenkelementen. Gegenstand ist auch die prophylaktische und/oder therapeutische Verwendung der erfindungsgemäßen Pelotte in einer Kniegelenksorthese zur Unterstützung der biomechanischen Funktion des infrapatellaren Fettkörpers des Kniegelenks, also insbesondere deren gelenksunterstützenden Funktion.

Gegenstand der Offenbarung ist auch die prophylaktische und/oder therapeutische Verwendung der erfindungsgemäßen Pelotte in einer Kniegelenksorthese zur Stabilisierung der Patella des Kniegelenks in Streckstellung.

Gegenstand der Offenbarung ist auch die prophylaktische und/oder therapeutische Verwendung der erfindungsgemäßen Pelotte in einer Kniegelenksorthese zur Behandlung des Vorderen Knieschmerzsyndroms.

Gegenstand der Offenbarung ist auch die prophylaktische und/oder therapeutische Verwendung der erfindungsgemäßen Pelotte in einer Kniegelenksorthese zur Behandlung einer Ödembildung in den infrapatellaren Fettkörpern des Kniegelenks.

Gegenstand der Offenbarung ist auch ein Verfahren zur Herstellung eines erfindungsgemäßen Elements, bei dem zwei Materialien unterschiedlicher Shorhärte nacheinander in ein Werkzeug eingespritzt oder gegossen werden. Einem Fachmann sind geeignete Werkzeuge und Verfahrensparameter aus der Gusstechnik und Spritzgusstechnik bekannt.

Gegenstand der vorliegenden Offenbarung ist auch die Verwendung von zwei Materialien unterschiedlicher Shorehärte zur Herstellung eines erfindungsgemäßen orthopädischen Medizinprodukts oder eines erfindungsgemäßen orthopädischen Hilfsmittels zur Behandlung der vorstehend genannten Krankheitsbilder.

Die Erfindung wird anhand der nachfolgenden Figuren näher beschrieben, ohne dass die dort dargestellten Ausführungsformen der Erfindung beschränkend zu verstehen sind.
Figur 1 zeigt einen Ausschnitt aus einem erfindungsgemäßen Element im Querschnitt;
Figur 2 zeigt den Querschnitt und eine Teilaufsicht einer erfindungsgemäßen Einlegesohle;
Figur 3 zeigt eine Totalansicht der erfindungsgemäßen Einlegesohle aus Figur 2.

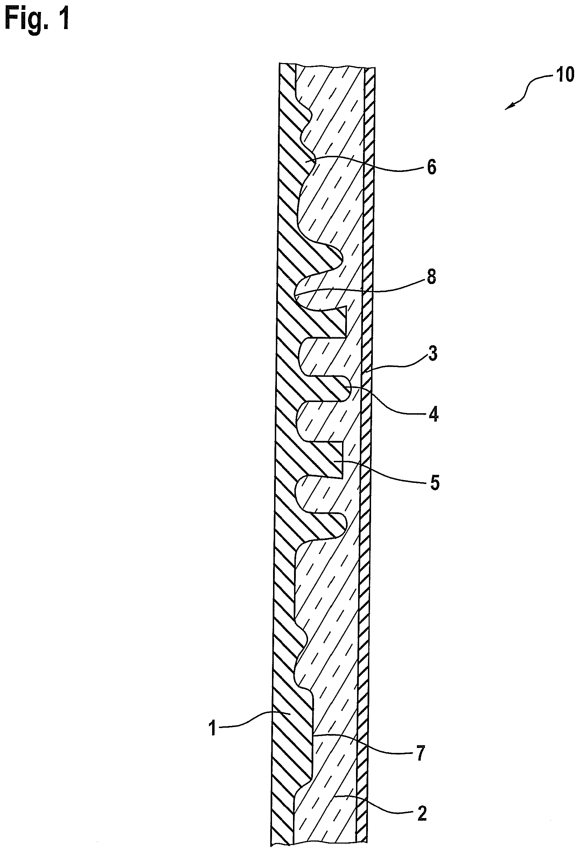

Figur 1 zeigt einen Ausschnitt aus einem erfindungsgemäßen Element (10) im Querschnitt. Das erfindungsgemäße Element (10) ist aus einer ersten schichtförmigen Komponente (1) und einer zweiten schichtförmigen Komponente (2) aufgebaut. Die erste Komponente (1) und die zweite Komponente (2) bestehen jeweils aus einem viscoelastischen Material. Die erste Komponente (1) ist aus einem härteren viscoelastischen Material gefertigt als die zweite Komponente (2). Zusätzlich ist auf der zweiten schichtförmigen Komponente (2) eine fakultative dritte schichtförmige Komponente (3) aufgebracht.

Die mit der zweiten Komponente (2) verbundene Fläche der ersten Komponenten (1) weist unterschiedliche Erhebungen (4,5,6,7) auf. In einem Bereich des Ausschnitts sind die Erhebungen (4,5) vergleichsweise hoch. Durch die hohen Erhebungen (4,5) der ersten Komponente (1) enthält diese Zone vergleichsweise mehr von dem härteren Material der ersten Komponente (1). Dadurch ist diese Zone des Elements (10) härter beziehungsweise weist eine geringere Viscoelastizität auf als eine Zone, in der keine oder nur vergleichsweise niedrige Erhebungen (6,7) vorhanden sind.

Die Erhebungen können unterschiedliche Formen haben. Beispielsweise können zapfenförmige Erhebungen (4,5,6) vorgesehen sein, wobei sich die Form der Zapfen unterscheiden kann, beispielsweise im Querschnitt runde Zapfen (4) oder im Querschnitt eckige (5) vorgesehen sein können. Alternativ können zu den Zapfen auch wellenförmige Erhebungen (7) vorgesehen sein.

Nicht nur durch die Höhe der Erhebungen (4,5,6,7), sondern auch durch die Form und Anzahl der Erhebungen kann die Härte beziehungsweise Viscoelastizität einer bestimmten Zone des Elements (10) bestimmt werden. So ist durch die wellenförmige Erhebung (7) in der dortigen Zone mehr härteres Material der ersten Komponente (1) vorhanden als in der Zone der zapfenförmigen Erhebung (6), so dass die Zone um die Erhebung (7) härter beziehungsweise weniger viscoelastisch ist als die Zone um die Erhebung (6).

Die Fläche der zweiten Komponente (2), die mit der ersten Komponente (1) verbunden ist, stellt ein Negativ der Fläche der ersten Komponente (1), die mit der zweiten Komponente (2) verbunden ist dar. So weist die zweite Komponente (2) an Stellen wo die erste Komponente (1) keine Erhebungen aufweist, Erhebungen (8) auf.

Die Vielzahl von Erhebungen (4,5,6,7) führt zu einer deutlichen Vergrößerung der Grenzfläche zwischen der ersten Komponente (1) und der zweiten Komponente (2). Darüber hinaus ist die Grenzfläche nicht plan. Dies führt zu einer verbesserten Verbindung der ersten Komponente (1) mit der zweiten Komponente (2).

Eine fakultative dritte Komponente (3) kann zum Beispiel auf der Fläche des Elements aufgebracht werden, die an dem Körper des Patienten anliegt. Sie kann beispielsweise aus Stoff bestehen und somit zu einem besseren Tragegefühl beitragen.

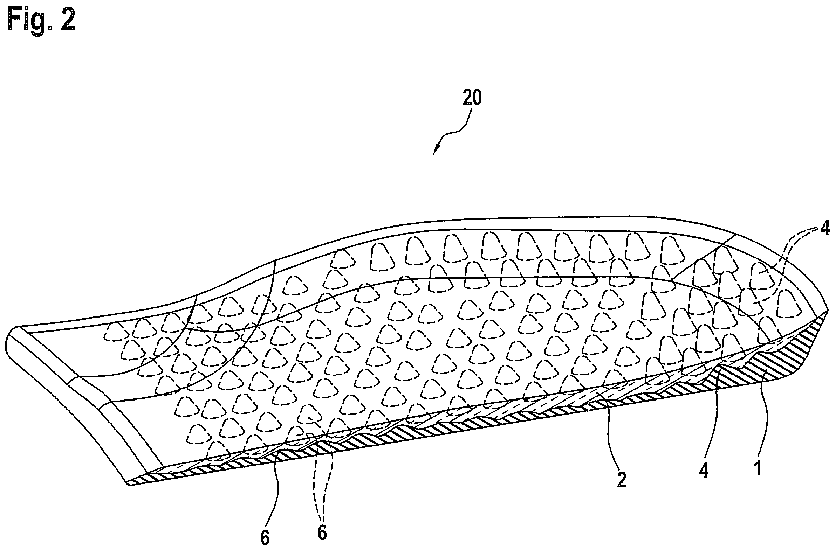

Figur 2 zeigt den Querschnitt und eine Teilansicht einer erfindungsgemäßen Einlegesohle (20). Die Einlegesohle besteht aus einer härteren weniger viscoelastischen ersten Komponente (1) und einer weicheren mehr viscoelastischen zweiten Komponente (2). Der erfindungsgemäße Grundaufbau und die daraus resultierenden Vorteile und Abwandlungsmöglichkeiten basieren auf dem erfindungsgemäßen Element aus Figur 1.

Die härtere erste Komponente (1) ist unten, also in Richtung Schuh angeordnet, und die weichere zweite Komponente (2) ist oben, also Richtung Fuß angeordnet. Die weichere zweite Komponente (2) führt dabei als Auflagefläche des Fußes zu einem angenehmen Tragegefühl. Bei Bedarf kann aber diese Anordnung auch umgekehrt werden.

Die zweite Komponente (2) besteht aus einem durchscheinenden oder durchsichtigen Material. Daher ist der Gesamtaufbau der Einlegesohle (20) und insbesondere die Grenzfläche zwischen erster Komponente (1) und zweiter Komponente (2) sichtbar.

Die erste Komponente (1) weist eine Vielzahl von Erhebungen (4,6) auf. Dabei sind Flächenzonen vorgesehen, die eine hohe Dichte von hohen zapfenförmigen Erhebungen (4) aufweisen und somit härter, weniger viscoelastisch und damit stabiler sind, und Flächenzonen vorgesehen, die eine geringere Dichte von niedrigen zapfenförmigen Erhebungen (6) aufweisen, und somit weicher und mehr viscoelastisch sind. Die härteren Zonen mit den höheren Erhebungen (4) dienen beispielsweise der Stützung des Fußes, die Zonen mit niedrigen Erhebungen (6) stellen entlastende Bereiche der Einlegesohle dar. Darüber hinaus können höhere Erhebungen auch als Propriozeptionspunkte vorgesehen sein.

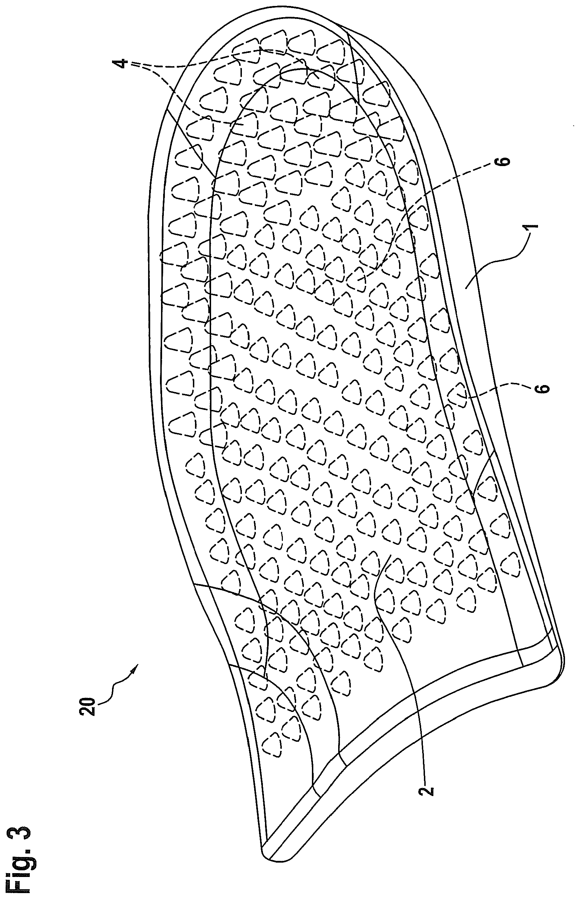

Figur 3 zeigt die Gesamtansicht der erfindungsgemäßen Einlegesohle (20) aus Figur 2. Diese besteht wieder aus einer härteren weniger viscoelastischen ersten Komponente (1), einer weicheren mehr viscoelastischen zweiten Komponente (2), wobei die erste Komponente eine Vielzahl von zapfenförmigen Erhebungen (4,6) aufweist.

## Patentansprüche

1. Element (10) für ein zumindest teilweise viscoelastisches orthopädisches Medizinprodukt oder für ein zumindest teilweise viscoelastisches orthopädisches Hilfsmittel, wobei das Element (10) mindestens eine erste schichtförmige Komponente (1) und eine zweite schichtförmige Komponente (2) enthält, wobei die erste Komponente (1) härter ist als die zweite Komponente (2), wobei eine Fläche der ersten Komponente (1) mit einer Fläche der zweiten Komponente (2) verbunden ist und wobei das Element (10) über seine Fläche mindestens zwei Zonen unterschiedlicher Härte aufweist, wobei die mit der zweiten Komponente (2) verbundene Fläche der ersten Komponente (1) eine Vielzahl von Erhebungen (4,5,6,7) aufweist, wobei über die Höhe, Breite und/oder die Verteilung der Erhebungen (4,5,6,7) in einer Zone des Elements (10) die Härte dieser Zonen bestimmt wird und wobei die Fläche der zweiten Komponente (2) an den Positionen der Erhebungen (4,5,6,7) der ersten Komponente(1) dementsprechende Vertiefungen aufweist, **dadurch gekennzeichnet, dass** die Oberflächen der ersten und der zweiten Komponente (1,2), die nicht mit der jeweils anderen Komponente verbunden sind, glatt sind.

2. Element (10) nach Anspruch 1, wobei die Schichtdicke der ersten Komponente (1) im Bereich der Erhebungen (4,5,6,7) größer ist und wobei die Schichtdicke der zweiten Komponente (2) im Bereich der Erhebungen (4,5,6,7) kleiner ist.

3. Element (10) nach einem der vorhergehenden Ansprüche, wobei es sich um eine Einlegesohle (20) oder ein Fersenkissen handelt.

4. Element (10) nach Anspruch 3, wobei die erste Komponente (1) den oberen oder unteren Teilbereich der Einlegesohle (20) oder des Fersenkissens bildet und die zweite Komponente (2) entsprechend den unteren oder oberen Teil der Einlegesohle (20) oder des Fersenkissens bildet.

5. Element (10) nach einem der Ansprüche 1 bis 2, wobei es sich um eine Pelotte handelt.

6. Element (10) nach einem der vorhergehenden Ansprüche, wobei die erste (1) und die zweite (2) Komponente aus Materialien mit unterschiedlicher Shorehärte bestehen.

7. Element (10) nach einem der vorhergehenden Ansprüche, wobei die Erhebungen (4,5,6,7) zapfenförmig oder wellenförmig sind.

8. Element (10) nach einem der vorhergehenden Ansprüche, wobei die erste Komponente (1) eine Vielzahl von Zapfen als Erhebungen (4,5,6,7) aufweist.

9. Element (10) nach einem der Ansprüche 7 oder 8, wobei die Zapfen einen runden (4), ovalen, ellipsoiden oder mehreckigen (5) Querschnitt haben.

10. Element (10) nach einem der vorhergehenden Ansprüche, wobei die erste Komponente (1) und/oder die zweite Komponente (2) aus einem durchsichtigen oder durchscheinenden Material bestehen.

11. Element (10) nach einem der vorhergehenden Ansprüche, wobei das Element (10) zusätzlich mindestens eine dritte Komponente (3) aufweist.

12. Element (10) nach Anspruch 11, wobei die dritte Komponente (3) in einem Teilbereich der ersten Komponente (1) und/oder der zweiten Komponente (2) durch die durch die erste Komponente (1) und/oder die zweite Komponente (2) gebildete Schicht hindurchreicht oder in die durch die erste Komponente (1) und/oder die zweite Komponente (2) gebildete Schicht eingebettet ist.

13. Orthopädisches Medizinprodukt oder orthopädisches Hilfsmittel, enthaltend ein Element (10) nach einem der Ansprüche 1 bis 12.

14. Orthopädisches Medizinprodukt oder orthopädisches Hilfsmittel nach Anspruch 13, wobei es sich um eine Einlegesohle (20), ein Fersenkissen oder eine Pelotte handelt.

## Claims

1. Element (10) for an at least partially viscoelastic orthopedic medical product, or for an at least partially viscoelastic orthopedic aid, wherein the element (10) comprises at least one first layered component (1) and a second layered component (2), wherein the first component (1) is harder than the second component (2), wherein a surface of the first component (1) is connected to a surface of the second component (2); and wherein said element (10) comprises at least two zones of different hardness on its surface, wherein the surface of the first component (1) connected to the second component (2) has a plurality of protrusions (4,5,6,7), wherein the hardness of these zones is determined by the height, the width, and/or the distribution of the protrusions (4,5,6,7) in a zone of the element (10), and wherein the surface of the second component (2) at the positions of the protrusions (4,5,6,7) of the first component (1) has corresponding depressions, **characterized in that** the surfaces of the first and second components (1,2) which are not bonded to each other are smooth.

2. Element (10) according to claim 1, wherein the layer thickness of the first component (1) is greater in the region of the protrusions (4,5,6,7) and wherein the layer thickness of the second component (2) is smaller in the region of the protrusions (4,5,6,7).

3. Element (10) according to any one of the preceding claims, wherein the element is an insole (20) or a heel cushion.

4. Element (10) according to claim 3, wherein the first component (1) forms the upper or lower portion of the insole (20) or of the heel cushion, and wherein the second component (2) correspondingly forms the lower or upper part of the insole (20) or of the heel cushion.

5. Element (10) according to any one of claims 1 to 2, wherein the element is a pad.

6. Element (10) according to any one of the preceding claims, wherein the first component (1) and the second component (2) consist of materials of different Shore hardness.

7. Element (10) according to any one of the preceding claims, wherein the protrusions (4,5,6,7) are peg-like or wave-like protrusions.

8. Element (10) according to any one of the preceding claims, wherein the first component (1) comprises a plurality of pegs as protrusions (4,5,6,7).

9. Element (10) according to any one of claims 7 or 8, wherein the pegs have a round (4), an oval, an ellipsoidal, or a polygonal (5) cross-section.

10. Element (10) according to any one of the preceding claims, wherein the first component (1) and/or the second component (2) consists of a transparent or a translucent material.

11. Element (10) according to any one of the preceding claims, wherein the element (10) further comprises at least a third component (3).

12. Element (10) according to claim 11, wherein, in a partial region of the first component (1) and/or the second component (2), the third component (3) extends through a layer formed by the first component (1) and/or the second component (2), or is embedded in the layer formed by the first component (1) and/or the second component (2).

13. Orthopedic medical product or orthopedic aid comprising an element (10) according to any one of claims 1 to 12.

14. Orthopedic medical product or orthopedic aid according to claim 13, wherein the orthopedic medical product or the orthopedic aid is an insole (20), a heel cushion, or a pad.

## Revendications

1. Élément (10) pour un produit médical orthopédique au moins partiellement viscoélastique ou pour un appareillage orthopédique au moins partiellement viscoélastique, l'élément (10) contenant au moins un premier composant (1) en forme de couche et un deuxième composant (2) en forme de couche, le premier composant (1) étant plus dur que le deuxième composant (2), une face du premier composant (1) étant reliée à une face du deuxième composant (2) et l'élément (10) présentant, sur sa face, au moins deux zones de duretés différentes, la face du premier composant (1), reliée au deuxième composant (2), présentant une pluralité de surélévations (4,5,6,7), la dureté de ces zones étant déterminée par la hauteur, la largeur et/ou la répartition des surélévations (4,5,6,7) dans une zone de l'élément (10), et la face du deuxième composant (2) présentant, aux positions des surélévations (4,5,6,7) du premier composant (1), des renfoncements correspondants, **caractérisé en ce que** les surfaces du premier et du deuxième composant (1,2) qui ne sont pas respectivement reliées à l'autre composant, sont lisses.

2. Élément (10) selon la revendication 1, l'épaisseur de couche du premier composant (1) étant plus grande dans la zone des surélévations (4,5,6,7) et l'épaisseur de couche du deuxième composant (2) étant plus petite dans la zone des surélévations (4,5,6,7).

3. Élément (10) selon l'une des revendications précédentes, l'élément en question étant une semelle intérieure (20) ou une talonnette.

4. Élément (10) selon la revendication 3, le premier composant (1) formant la partie supérieure ou inférieure de la semelle intérieure (20) ou de la talonnette et le deuxième composant (2) formant en conséquence la partie inférieure ou supérieure de la semelle intérieure (20) ou de la talonnette.

5. Élément (10) selon l'une des revendications 1 à 2, l'élément en question étant une pelote.

6. Élément (10) selon l'une des revendications précédentes, le premier (1) et le deuxième (2) composant étant composés de matières présentant des duretés Shore différentes.

7. Élément (10) selon l'une des revendications précédentes, les surélévations (4,5,6,7) étant de forme conique ou de forme ondulée.

8. Élément (10) selon l'une des revendications précédentes, le premier composant (1) présentant une pluralité de cônes comme surélévations (4,5,6,7).

9. Élément (10) selon l'une des revendications 7 ou 8, les cônes ayant une section ronde (4), ovale, ellipsoïde ou polygonale (5).

10. Élément (10) selon l'une des revendications précédentes, le premier composant (1) et/ou le deuxième composant (2) étant composés d'une matière transparente ou translucide.

11. Élément (10) selon l'une des revendications précédentes, l'élément (10) présentant en plus au moins un troisième composant (3).

12. Élément (10) selon la revendication 11, le troisième composant (3) traversant une partie du premier composant (1) et/ou du deuxième composant (2) en passant par la couche formée par le premier composant (1) et/ou le deuxième composant (2) ou bien étant intégré dans la couche formée par le premier composant (1) et/ou le deuxième composant (2).

13. Produit médical orthopédique ou appareillage orthopédique, contenant un élément (10) selon l'une des revendications 1 à 12.

14. Produit médical orthopédique ou appareillage orthopédique selon la revendication 13, le produit ou l'appareillage en question étant une semelle intérieure (20), une talonnette ou une pelote.
